(19) Europäisches Patentamt
European Patent Office
Office européen des brevets

(11) EP 0 770 601 A1

(12) EUROPEAN PATENT APPLICATION
published in accordance with Art. 158(3) EPC

(43) Date of publication:
**02.05.1997 Bulletin 1997/18**

(21) Application number: **96912261.3**

(22) Date of filing: **26.04.1996**

(51) Int. Cl.[6]: **C07D 209/12**, C07D 405/10, A61K 31/40

(86) International application number:
**PCT/JP96/01156**

(87) International publication number:
**WO 96/35665 (14.11.1996 Gazette 1996/50)**

(84) Designated Contracting States:
**AT BE CH DE DK ES FI FR GB GR IE IT LI LU MC NL PT SE**

(30) Priority: **10.05.1995 JP 111741/95**

(71) Applicant: **Kyowa Hakko Kogyo Co., Ltd.**
**Chiyoda-ku, Tokyo 100 (JP)**

(72) Inventors:
- **IKEDA, Shun-ichi**
**Machida-shi, Tokyo 194 (JP)**
- **KANAZAWA, Junji**
**Sunto-gun, Shizuoka 411 (JP)**
- **GOMI, Katsushige**
**Shizuoka 410-11 (JP)**
- **SAITO, Hiromitsu**
**Kawasaki-shi, Kanagawa 214 (JP)**
- **ASHIZAWA, Tadashi**
**Numazu-shi, Shizuoka 410 (JP)**

(74) Representative: **VOSSIUS & PARTNER**
**Postfach 86 07 67**
**81634 München (DE)**

(54) **PROPENONE DERIVATIVES**

(57) The present invention relates to propenone derivatives represented by the following formula (I):

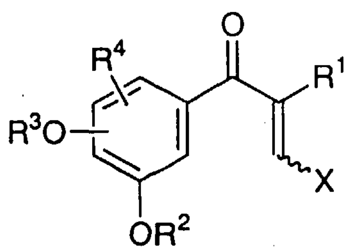

(I)

wherein $R^1$ represents hydrogen or lower alkyl; $R^2$ and $R^3$ independently represent hydrogen, lower alkyl, or substituted or unsubstituted aralkyl, or alternatively $R^2$ and $R^3$ are combined to form substituted or unsubstituted methylene or ethylene; $R^4$ represents hydrogen, hydroxy, lower alkyl, substituted or unsubstituted aralkyl, lower alkoxy, substituted or unsubstituted aralkyloxy, or halogen; and X represents substituted or unsubstituted indolyl, with the proviso that when $OR^3$ is on the position 2 or position 6 of the benzene ring, $R^3$ is not hydrogen; that when $R^4$ is on the position 2 or position 6 of the benzene ring, $R^4$ is not hydroxy; and that the combination of $OR^2$, $OR^3$, and $R^4$ is not 3,4-dimethoxy or 3,4,5-trimethoxy; or pharmaceutically acceptable salts thereof.

EP 0 770 601 A1

## Description

### Technical Field

The present invention relates to propenone derivatives having an antitumor activity.

### Background Art

Typical examples of compounds having an antitumor activity include mitomycin C, adriamycin, vincristine, and the like, all of which are clinically used as useful anticancer agents. However, since each of the compounds also has adverse effects such as myelotoxicity, cardiotoxicity, nerve damage, etc., a novel anticancer agent having less adverse effects is demanded.

Chalcone derivatives are known as having the activity to inhibit polymerization of tubulin [Journal of the Medicinal Chemistry (J. Med. Chem.), 33, 1948 (1990) and Journal of Natural Products (J. Nat. Prod.), 56, 1718 (1993)]. Chalcone derivatives are also known as having an anticancer activity (U. S. Patent No. 4904697). 3-Indolyl-1-phenyl-2-propen-1-one derivatives are known as inhibiting the tyrosine-phospholylation of cell growth factor receptor [Cancer Research (Cancer Res.), 54, 6106 (1994) and WO 91/16305], being useful as an organic nonlinear optical material (Japanese Published Unexamined Patent Application No. 255426/91), and having an antiallergic activity [Khim.-Farm. Zh., 25, 18 (1991)].

Further, 3-(indol-3-yl)-1-phenyl-2-propen-1-one derivatives are disclosed in French Patent No. 2230349, Khim. Geterotsikl. Soedin, 1066 (1970), Khim. Geterotsikl. Soedin, 268 (1969), Khim. Geterotsikl. Soedin, 399 (1970), Farmaco Ed. Sci., 26, 591 (1971), etc.

### Disclosure of the Invention

The present invention relates to propenone derivatives represented by the following formula (I):

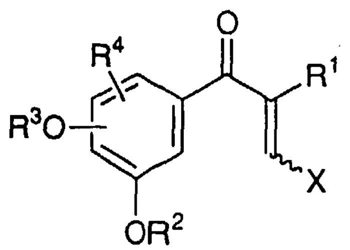

(I)

wherein $R^1$ represents hydrogen or lower alkyl; $R^2$ and $R^3$ independently represent hydrogen, lower alkyl, or substituted or unsubstituted aralkyl, or alternatively $R^2$ and $R^3$ are combined to form substituted or unsubstituted methylene or ethylene; $R^4$ represents hydrogen, hydroxy, lower alkyl, substituted or unsubstituted aralkyl, lower alkoxy, substituted or unsubstituted aralkyloxy, or halogen; and X represents substituted or unsubstituted indolyl, with the proviso that when $OR^3$ is on the position 2 or position 6 of the benzene ring, $R^3$ is not hydrogen; that when $R^4$ is on the position 2 or position 6 of the benzene ring, $R^4$ is not hydroxy; and that the combination of $OR^2$, $OR^3$, and $R^4$ is not 3,4-dimethoxy or 3,4,5-trimethoxy; or pharmaceutically acceptable salts thereof.

Compounds represented by the formula (I) are hereinafter referred to as Compounds (I). Compounds (Ia) and the like are included in Compounds (I).

In the definitions of the groups of Compounds (I), the lower alkyl and the lower alkyl moiety of the lower alkoxy mean a straight-chain or branched alkyl group having 1 to 6 carbon atoms, such as methyl, ethyl, propyl, isopropyl, butyl, 2-butyl, isobutyl, tert-butyl, 1-pentyl, 2-pentyl, 3-pentyl, isoamyl, neopentyl, and hexyl. The aralkyl and the aralkyl moiety of the aralkyloxy mean an aralkyl group having 7 to 15 carbon atoms, such as benzyl, naphthylmethyl, and benzhydryl. The halogen includes fluorine, chorine, bromide, and iodine.

The substituted aralkyl and substituted aralkyloxy each has the same or different 1 to 3 substituents such as lower alkyl, hydroxy, lower alkoxy, amino, lower alkylamino, di(lower alkyl)amino, lower alkanoylamino, lower alkoxycarbonylamino, halogen, nitro, carboxy, lower alkanoyl, and lower alkoxycarbonyl. In the definitions of the substituents, the lower alkyl moiety of the lower alkylamino, di(lower alkyl)amino, lower alkanoylamino, lower alkoxycarbonylamino, lower alkanoyl, and lower alkoxycarbonyl has the same meaning as the lower alkyl defined above, and the lower alkyl, lower alkoxy, and halogen have the same meanings as defined above.

The substituted methylene or ethylene has the same or different 1 to 3 substituents such as lower alkyl, and the lower alkyl has the same meaning as defined above.

Examples of the substituent on the nitrogen atom at position 1 of the substituted indolyl group are lower alkyl, lower

alkanoyl, lower alkoxycarbonyl, and aralkyl, and examples of the substituents on the carbon atoms at positions 2 to 7 of the substituted indolyl group are lower alkyl, lower alkoxy, amino, lower alkylamino, di(lower alkyl)amino, lower alkanoylamino, lower alkoxycarbonylamino, halogen, nitro, carboxy, lower alkanoyl, lower alkoxycarbonyl, and aralkyl. In the definitions of the substituents, the lower alkyl, lower alkoxy, lower alkylamino, di(lower alkyl)amino, lower alkanoylamino, lower alkoxycarbonylamino, halogen, lower alkanoyl, lower alkoxycarbonyl, and aralkyl have the same meanings as defined above.

The pharmaceutically acceptable salts of Compounds (I) include inorganic acid addition salts such as hydrochloride, sulfate, and phosphate, organic acid addition salts such as acetate, maleate, fumarate, succinate, tartrate, citrate, oxalate, and methanesulfonate, alkali metal salts such as sodium salt and potassium salt, alkaline earth metal salts such as magnesium salt and calcium salt, metal salts such as aluminium salt and zinc salt, and ammonium salt.

The present invention is described in detail below.

In the processes shown below, if the defined groups are converted into undesired groups under the conditions of the processes or are not suitable for carrying out the processes, the processes can be readily carried out by applying thereto means conventionally used in organic synthetic chemistry, for example, a means such as protection or deprotection of functional groups, or a method such as oxidation, reduction, or hydrolysis.

Process for Producing Compound (I) - 1

Compound (I) can be prepared according to the following reaction step.

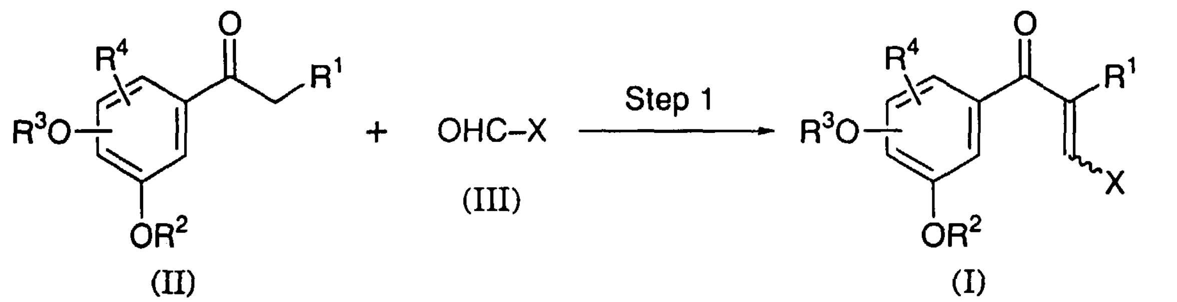


(In the formulae, $R^1$, $R^2$, $R^3$, $R^4$, and X have the same meanings as defined above.)

Step 1

Compound (I) can be obtained by reacting Compound (II) with Compound (III) in the presence of a base in an inert solvent. As the base, inorganic bases such as potassium carbonate, sodium carbonate, and cesium fluoride, quaternary ammonium fluorides such as tetra-n-butylammonium fluoride, secondary amines such as piperidine, pyrrolidine, and morpholine, metal alkoxides such as potassium tert-butoxide, metal amides such as lithium diisopropylamide, metal hydrides such as sodium hydride, and the like may be used in an amount of 0.01 to 10 equivalents. As the solvent, aprotic solvents (for example, ethyl acetate and tetrahydrofuran), aromatic hydrocarbons (for example, toluene), halogenated hydrocarbons (for example, chloroform), alcohols (for example, methanol and ethanol), and the like may be used alone or in combination. The reaction is carried out at the temperature between -78°C and the boiling point of the solvent employed in the reaction, and is completed in 0.1 hour to 10 days.

The starting Compound (II) is commercially available, is reported in a literature, or can be prepared according to the following reaction steps.

Process for Producing Compound (II) - 1

Compound (II) can be prepared according to the following reaction steps.

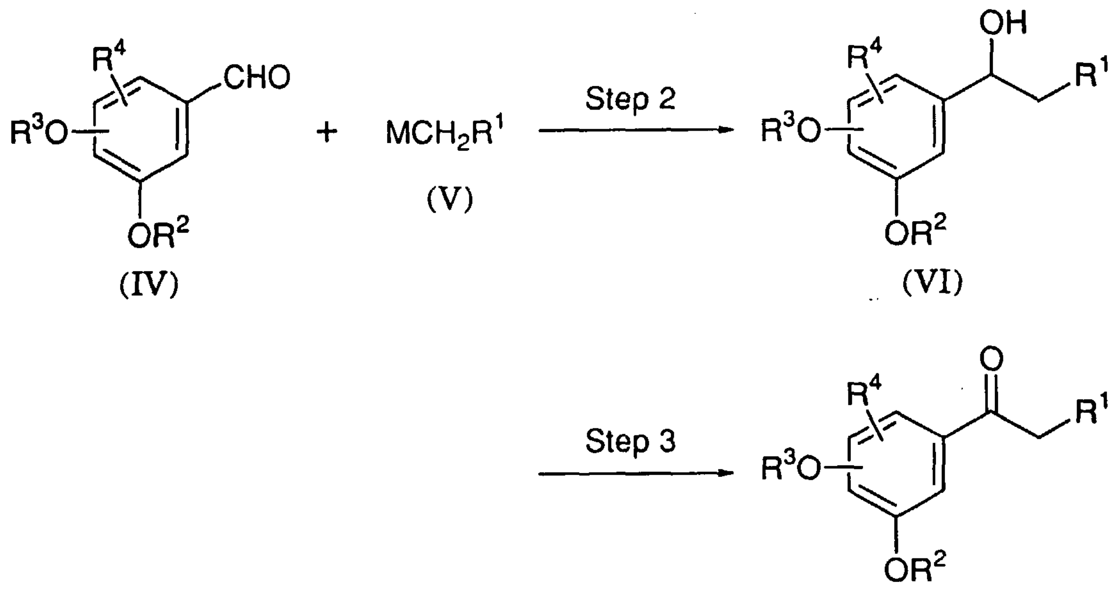


(IV) + $MCH_2R^1$ (V) → (VI) → (II)

(In the formulae, M represents alkali metal, alkaline earth metal halide, or cerium dichloride; and $R^1$, $R^2$, $R^3$, and $R^4$ have the same meanings as defined above.)

In the definition of M, the alkali metal means lithium, sodium, potassium, cesium, or the like, and the alkaline earth metal halide means magnesium chloride, magnesium bromide, magnesium iodide, or the like.

Step 2

Compound (VI) can be obtained by reacting Compound (IV) with 1 to 2 equivalents of Compound (V) in an inert solvent. As the solvent, aprotic solvents (for example, diethyl ether, tetrahydrofuran, and ethyl acetate), aromatic hydrocarbons (for example, toluene), and the like may be used alone or in combination. The reaction is carried out at the temperature between -100°C and the boiling point of the solvent employed in the reaction, and is completed in 0.1 to 24 hours.

Step 3

Compound (II) can be obtained by treating Compound (VI) in the presence of an oxidizing agent in an inert solvent. As the oxidizing agent, 1 to 50 equivalents of chromium trioxide, a pyridine complex or hydrochloric acid complex thereof, potassium dichromate, manganese dioxide, 2,3-dichloro-5,6-dicyanobenzoquinone, and the like may be used. As the solvent, aprotic solvents (for example, acetone and N,N-dimethylformamide), halogenated hydrocarbons (for example, dichloromethane and chloroform), acetic acid, sulfuric acid, water, and the like may be used alone or in combination. The reaction is carried out at the temperature between -10°C and the boiling point of the solvent employed in the reaction, and is completed in 0.1 to 150 hours.

Compound (IV) is commercially available, is reported in a literature, or can be prepared according to the following reaction steps.

Process for Producing Compound (IV) - 1

Compound (IVa) which is Compound (IV) in which $R^2$ is lower alkyl or substituted or unsubstituted aralkyl can be prepared according to the following reaction step from the aldehyde which is commercially available or known in the literature.

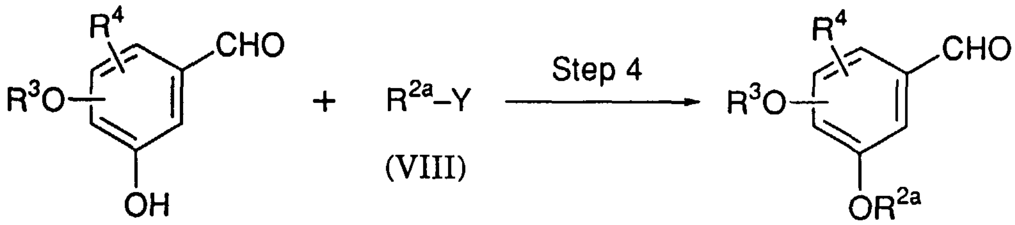


(VII) (VIII) (IVa)

(In the formulae, $R^{2a}$ represents lower alkyl or substituted or unsubstituted aralkyl; Y represents halogen; and $R^3$ and $R^4$ have the same meanings as defined above.)

In the definition of $R^{2a}$, the lower alkyl and substituted or unsubstituted aralkyl have the same meanings as defined above. In the definition of Y, the halogen has the same meaning as defined above.

Step 4

Compound (IVa) can be obtained by reacting Compound (VII) with Compound (VIII) in the presence of a base in an inert solvent. As the base, inorganic bases such as sodium hydroxide, potassium carbonate, sodium carbonate, and cesium fluoride, quaternary ammonium fluorides such as tetra-n-butylammonium fluoride, metal alkoxides such as potassium tert-butoxide, metal amides such as lithium diisopropylamide, metal hydrides such as sodium hydride, and the like may be used in an amount of 1 to 100 equivalents. As the solvent, aprotic solvents (for example, ethyl acetate, tetrahydrofuran, acetone, and N,N-dimethylformamide), aromatic hydrocarbons (for example, toluene), halogenated hydrocarbons (for example, chloroform), alcohols (for example, methanol and ethanol), water, and the like may be used alone or in combination. The reaction is carried out at the temperature between -78°C and the boiling point of the solvent employed in the reaction, and is completed in 0.1 hour to 3 days.

Process for Producing Compound (IV) - 2

Compound (IVb) which is Compound (IV) in which $R^3$ is lower alkyl or substituted or unsubstituted aralkyl can be prepared according to the following reaction step from the aldehyde which is commercially available or known in the literature.

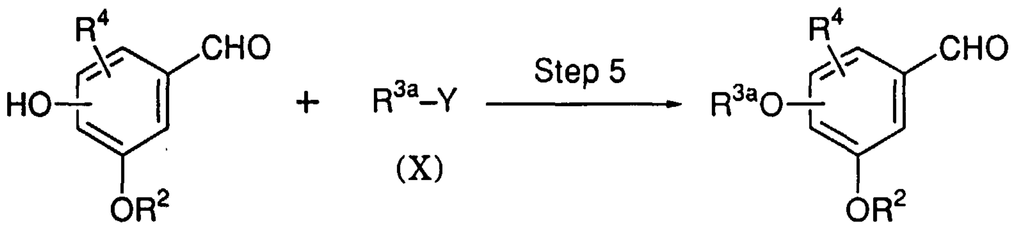


(IX) (X) (IVb)

(In the formulae, $R^{3a}$ represents lower alkyl or substituted or unsubstituted aralkyl; and $R^2$, $R^4$, and Y have the same meanings as defined above.)

Step 5

Compound (IVb) can be obtained by reacting Compound (IX) with Compound (X) according to the same method in Step 4.

Process for Producing Compound (IV) - 3

Compound (IV) can be prepared according to the following reaction steps from the carboxylic acid or ester which is commercially available or known in the literature.

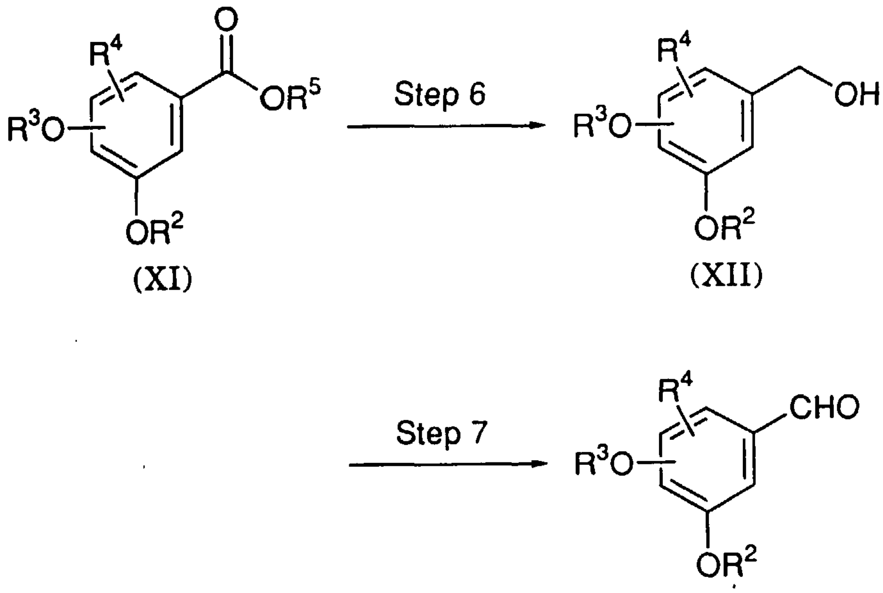


(XI) (XII)

(IV)

(In the formulae, $R^5$ represents hydrogen, lower alkyl, or aralkyl; and $R^2$, $R^3$, and $R^4$ have the same meanings as defined above.)

In the definition of $R^5$, the lower alkyl and aralkyl have the same meanings as defined above.

Step 6

Compound (XII) can be obtained by treating Compound (XI) with a hydride reagent in an inert solvent. As the hydride reagent, 1 to 100 equivalents of sodium borohydride, lithium aluminum hydride, alane, borane, diisopropyl aluminium hydride, and the like may be used. As the solvent, aprotic solvents (for example, diethyl ether and tetrahydrofuran), aromatic hydrocarbons (for example, toluene), alcohols (for example, methanol and ethanol), and the like may be used alone or in combination. The reaction is carried out at the temperature between -78°C and the boiling point of the solvent employed in the reaction, and is completed in 0.1 hour to 3 days.

Step 7

Compound (IV) can be obtained by reacting Compound (XII) according to the same method in Step 3.

Process for Producing Compound (II) - 2

Compound (II) can be prepared according to the following reaction step from the nitrile which is commercially available or known in the literature.

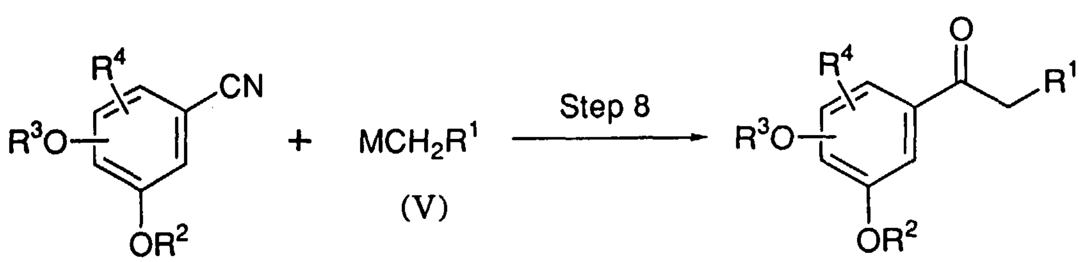


(XIII) (V) (II)

(In the formulae, $R^1$, $R^2$, $R^3$, $R^4$, and M have the same meanings as defined above.)

Step 8

Compound (II) can be obtained by reacting Compound (XIII) with Compound (V) according to the same method in Step 2.

Process for Producing Compound (II) - 3

Compound (IIa) which is Compound (II) in which $R^2$ is lower alkyl or substituted or unsubstituted aralkyl can be prepared according to the following reaction step from the ketone which is commercially available or known in the literature.

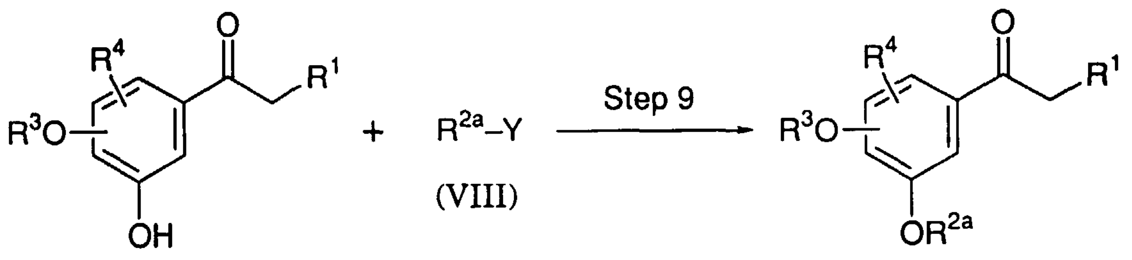


(XIV) (VIII) (IIa)

(In the formulae, $R^1$, $R^{2a}$, $R^3$, $R^4$, and Y have the same meanings as defined above.)

Step 9

Compound (IIa) can be obtained by reacting Compound (XIV) with Compound (VIII) according to the same method in Step 4.

Process for Producing Compound (II) - 4

Compound (IIb) which is Compound (II) in which $R^3$ is lower alkyl or substituted or unsubstituted aralkyl can be prepared according to the following reaction step from the ketone which is commercially available or known in the literature.

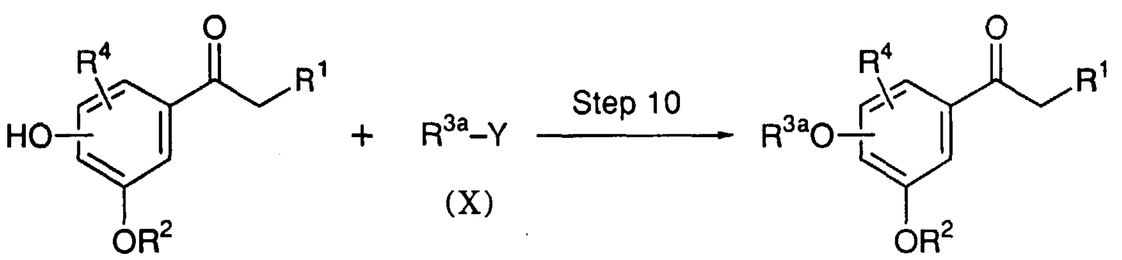


(XV) (X) (IIb)

(In the formulae, $R^1$, $R^2$, $R^{3a}$, $R^4$, and Y have the same meanings as defined above.)

Step 10

Compound (IIb) can be obtained by reacting Compound (XV) with Compound (X) according to the same method in Step 4.

Process for Producing Compound (I) - 2

Compound (I) can be prepared according to the following reaction steps.

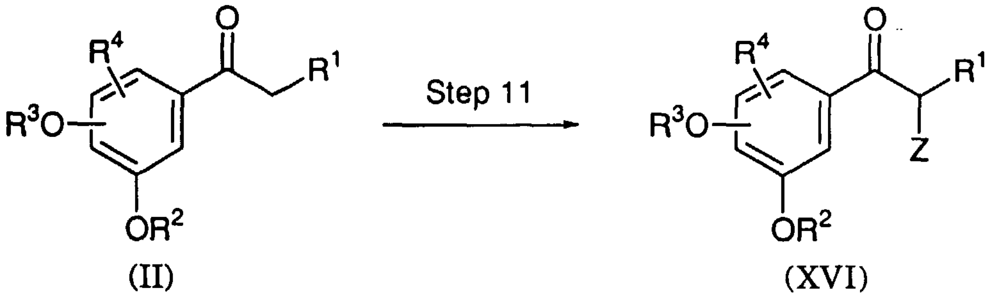


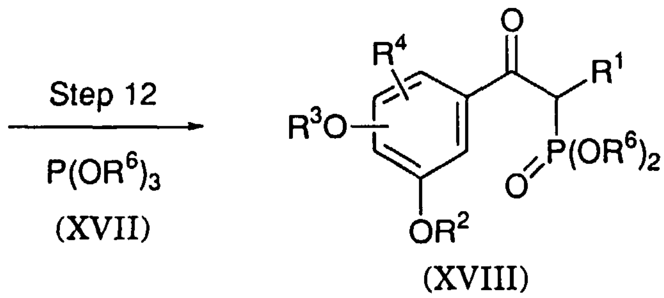


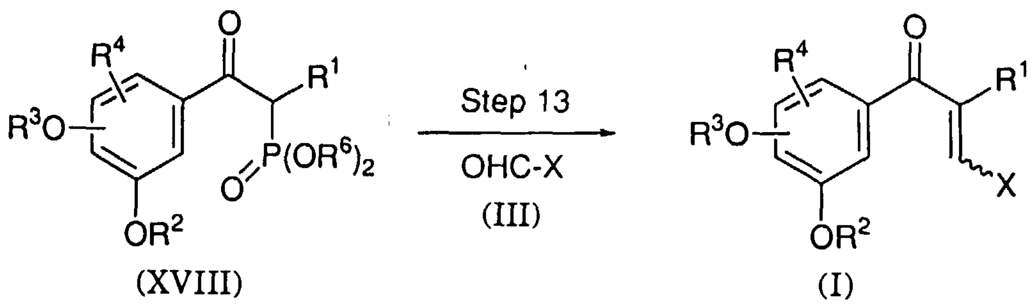


(In the formulae, $R^6$ represents lower alkyl; Z represents halogen; and $R^1$, $R^2$, $R^3$, $R^4$, and X have the same meanings as defined above.)

In the definition of $R^6$, the lower alkyl has the same meaning as defined above. In the definition of Z, the halogen has the same meaning as defined above.

Step 11

Compound (XVI) can be obtained by treating Compound (II) with a halogenating agent in an inert solvent. As the halogenating agent, 1 to 5 equivalents of pyrrolidone hydrotribromide, tetra-n-butylammonium tribromide, bromine, and the like may be used. As the solvent, aprotic solvents (for example, ethyl acetate and tetrahydrofuran), acetic acid, water, and the like may be used alone or in combination. The reaction is carried out at the temperature between 0°C and the boiling point of the solvent employed in the reaction, and is completed in 0.1 to 24 hours.

Step 12

Compound (XVIII) can be obtained by reacting Compound (XVI) with 1 to 10 equivalents of Compound (XVII) in an inert solvent or without a solvent. As the solvent, aprotic solvents (for example, ethyl acetate, tetrahydrofuran, and N,N-dimethylformamide), aromatic hydrocarbons (for example, toluene), and the like may be used alone or in combination. The reaction is carried out at the temperature between 0°C and 200°C, and is completed in 0.5 to 100 hours.

Step 13

Compound (I) can be obtained by reacting Compound (XVIII) with Compound (III) according to the same method in Step 1.

The starting Compound (III) is commercially available, is known in the literature, or can be prepared according to the reaction steps which is known in the literature.

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without isolation.

In the case where a salt of Compound (I) is desired and it is produced in the form of the desired salt, it can be subjected to purification as such. In the case where Compound (I) is produced in the free form and its salt is desired, Compound (I) is dissolved or suspended in a suitable solvent, followed by addition of an acid or base to form a salt by a conventional method.

Compounds (I) can exist in the form of E/Z geometrical isomers, and the present invention covers all isomers including these geometrical isomers and mixtures thereof. In the case where Compound (I) is obtained in a E/Z mixture, and separation of E/Z isomers is desired, they can be isolated and purified by fractionation methods, for example, fractional crystallization, fractional precipitation, fractional dissolution, or the like.

Compounds (I) and pharmaceutically acceptable salts thereof may be in the form of adducts with water or various solvents, which are also within the scope of the present invention.

Examples of Compounds (I) obtained in the processes described above are shown in Table 1.

Table 1-1

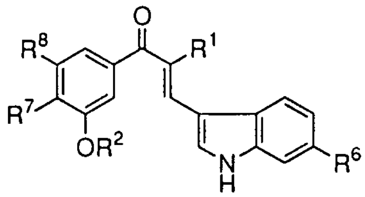

| Compd. No. | $R^8$ | $R^7$ | $OR^2$ | $R^1$ | $R^6$ |
|---|---|---|---|---|---|
| 1 | $OCH_3$ | H | $OCH_3$ | H | H |
| 2 | $OCH_3$ | OH | $OCH_3$ | H | H |
| 3 | $OCH_3$ | $OCH_2C_6H_5$ | $OCH_3$ | H | H |
| 4 | $OCH_2CH_3$ | $OCH_2CH_3$ | $OCH_2CH_3$ | $CH_3$ | H |
| 5 | $OCH_3$ | $OCH_2CH_3$ | $OCH_3$ | $CH_3$ | H |
| 6 | $OCH_3$ | $OCH_2CH(CH_3)_2$ | $OCH_3$ | $CH_3$ | H |
| 7 | $OCH_3$ | $CH_2CH_3$ | $OCH_3$ | $CH_3$ | H |
| 8 | $OCH_3$ | - O $CH_2$ O - | | $CH_3$ | H |
| 9 | $OCH_2CH_3$ | $OCH_3$ | $OCH_3$ | $CH_3$ | H |
| 10 | Br | $OCH_3$ | $OCH_3$ | $CH_3$ | H |
| 11 | $OCH_3$ | $OCH_2CH_3$ | $OCH_3$ | $CH_3$ | $CH_3$ |
| 12 | $OCH_3$ | - O $CH_2$ O - | | $CH_3$ | $CH_3$ |
| 13 | Br | $OCH_2CH(CH_3)_2$ | $OCH_3$ | $CH_3$ | H |
| 14 | Br | $OCH_2CH(CH_3)_2$ | $OCH_3$ | $CH_3$ | $CH_3$ |
| 15 | $OCH_3$ | $OCH_2CH(CH_3)_2$ | $OCH_3$ | $CH_3$ | $CH_3$ |
| 16 | $OCH_3$ | $OCH_2CH_3$ | $OCH_3$ | $CH_3$ | $CH_2CH_3$ |
| 17 | $OCH_3$ | $OCH_2CH_3$ | $OCH_3$ | $CH_3$ | $CH(CH_3)_2$ |
| 18 | $OCH_3$ | $OCH_2CH_3$ | $OCH_3$ | $CH_3$ | Cl |
| 19 | $OCH_3$ | $OCH_2CH_2CH_3$ | $OCH_3$ | $CH_3$ | $CH_3$ |
| 20 | $OCH_3$ | $O(CH_2)_3CH_3$ | $OCH_3$ | $CH_3$ | $CH_3$ |

Table 1-2

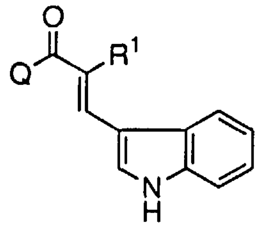

| Compd. No. | Q | $R^1$ |
|---|---|---|
| 21 | 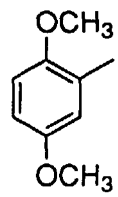 | H |
| 22 | 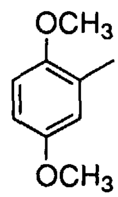 | $CH_3$ |
| 23 | 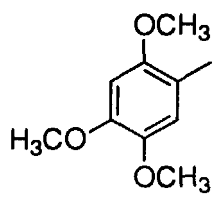 | $CH_3$ |
| 24 | 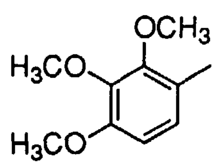 | $CH_3$ |

The antitumor activities of Compounds (I) are shown in detail below by test examples.

Test Example 1: HeLa $S_3$ Cell Growth Inhibition Test

Each 0.1 ml of HeLa $S_3$ cells which had been prepared to 3 x $10^4$ cells/ml using a medium consisted of MEM medium, 10% fetal bovine serum and 2 mM glutamine was distributed in each well of 96 well-microtiter plate. HeLa $S_3$ was cultured at 37°C in a $CO_2$ incubator for one night, each 0.05 ml of test compounds which had been appropriately diluted with the culture solution was added thereto and the mixture was cultured at 37°C for 72 hours in a $CO_2$ incubator. Supernatant was removed, each 0.1 ml of the culture solution containing 0.02% neutral red was added to the residue, the mixture was incubated at 37°C for one hour in a $CO_2$ incubator and the cells were stained. Supernatant was removed and the residue was washed once with physiological saline. Then, the pigment was extracted with 0.001 N hydrochloric acid/30% ethanol and the absorbance at 550 nm was measured by a microplatereader. A concentration of

the test compound ($IC_{50}$) at which the growth of cell is inhibited by 50% was calculated by comparing the absorbance of non-treated cells and that of cells treated with a predetermined concentration of the test compound.

The results are shown in Table 2.

Table 2

| Compd. No. | $IC_{50}$ (72 hours, nM) |
|---|---|
| 4 | 22 |
| 5 | 2.5 |
| 6 | 9.4 |
| 7 | 64 |
| 8 | 6.1 |
| 9 | 3.2 |
| 10 | 6.6 |
| 11 | 6.7 |
| 12 | 2.3 |
| 15 | 18 |
| 16 | 6.2 |
| 18 | 6.3 |
| 19 | 5.2 |
| 20 | 6.1 |
| 24 | 22 |

Test Example 2: Effect upon P388 Ascites Tumor

The experiment was carried out by using groups of 6-weeks-old male $CDF_1$ mice, each group consisting of five mice. $10^6$ cells of P388 mouse leukemia were implanted into the abdominal cavities of the mice. A test compound was sufficiently wetted by adding 10 μl of Tween 80 relative to 1 mg of a sample, and 0.3% CMC (sodium carboxymethyl cellulose) solution was then added to the test compound to form a suspension. The resultant suspension was administered once 24 hours after implantation of the tumor, or repeatedly for consecutive 5 days from 24 hours after implantation of the tumor. The average survival day (T) in a group was calculated from the survival days of the respective mice in the group administered with the test compound at each dose. On the other hand, the average survival day (C) of a group which was not administered was measured, and the increased life span [ILS (%)] was calculated according to the following equation:

$$[(T - C)/C] \times 100\ (\%)$$

The results are shown in Table 3.

Table 3

| Compd. No. | ILS (%) [Dose (mg/kg)] | |
|---|---|---|
| | five consec. admin. | single admin. |
| 4 | NT | 21 ( 50) |
| 5 | 41 ( 3.1) | 59 ( 25) |
| 6 | 43 ( 3.1) | 32 ( 6.3) |
| 7 | 40 ( 25) | 43 (100) |
| 8 | 50 ( 6.3) | 36 ( 50) |
| 9 | NT | 51 ( 25) |
| 10 | NT | 30 ( 25) |
| 11 | NT | 36 ( 13) |
| 12 | NT | 45 ( 6.3) |
| NT; not tested | | |

The compounds of the present invention are useful as antitumor agents, and can be used as they are or in various administration forms. For example, when Compounds (I) are used as injections, Compounds (I) may be dissolved in a diluting agent conventionally used in this field such as physiological saline, glucose injections, lactose injections, mannitol injections or the like, freeze-dried on the basis of the Japanese Pharmacopoeia, or mixed with sodium chloride to form powder injections. Compounds (I) can also be used as lipid emulsions. These injections may contain an adjuvant such as polyethylene glycol, HCO-60 (surfactant: produced by Nikko Chemical Co., Ltd.) or the like; and a carrier such as ethanol and/or liposome, cyclodextrin or the like. Although the injections are generally subjected to intravenous administration, they can also be subjected to arterial administration, intraabdominal administration or intrathoracic administration.

When Compounds (I) are mixed with appropriate excipients, disintegrators, binders, lubricants and the like and formed to tablets, granules, powders, syrups or the like by a conventional method, the compounds can also be used as oral agents. Compounds (I) may be mixed with carriers conventionally used and formed, by a conventional method, to suppositories which can be administered to the rectum.

The dose varies depending upon the mode of administration, the type of Compound (I), the age and conditions of a patient, etc., and the administration schedule can be changed according to the conditions of a patient or the dose. For example, intravenous administration can be made at a dose of 0.01 to 1000 mg/60 kg once a week or once every three weeks.

Examples are described below.

Best Mode for Carrying Out the Invention

The physicochemical data of each compound were measured by the following apparatus.

$^{1}$H-NMR: Nihon Denshi JNM-GX270 (270 MHz) Hitachi R-90H (90 MHz)
MS: Nihon Denshi JSM-D300
Elemental Analysis: Perkin Elmer 2400 CHN Analyzer

Example 1

(E)-1-(3,5-Dimethoxyphenyl)-3-(indol-3-yl)-2-propen-1-one (Compound 1)

3',5'-Dimethoxyacetophenone (1.10 g) and indole-3-carboxaldehyde (1.45 g) were dissolved in ethanol (20 ml), and piperidine (0.85 g) was added thereto, followed by heating under reflux for 32 hours. The reaction solution was cooled to room temperature, and the precipitated crystals were collected by filtration, followed by recrystallization from ethanol to give Compound 1 (1.79 g).

$^1$H-NMR (270 MHz, DMSO-$d_6$) δ 3.85 (s, 6H), 6.70 (t, J = 2.5 Hz, 1H), 7.19 (t, J = 2.5 Hz, 2H), 7.21-7.27 (m, 2H), 7.50 (m, 1H) 7.56 (d, J = 15.6 Hz, 1H), 8.04 (m, 1H), 8.05 (d, J = 15.6 Hz, 1H), 8.14 (d, J = 2.5 Hz, 1H), 11.91 (s, 1H)
EI-MS m/z = 307 ($M^+$)

| Elemental analysis: $C_{19}H_{17}NO_3$ | | | |
|---|---|---|---|
| Calcd.(%): | C, 74.25; | H, 5.58; | N, 4.56 |
| Found (%): | C, 74.15; | H, 5.79; | N, 4.24 |

## Example 2

(E)-1-(4-Hydroxy-3,5-dimethoxyphenyl)-3-(indol-3-yl)-2-propen-1-one (Compound 2)

4'-Hydroxy-3',5'-dimethoxyacetophenone (1.96 g) and indole-3-carboxaldehyde (1.45 g) were dissolved in ethanol (20 ml), and piperidine (0.85 g) was added thereto, followed by heating under reflux for 32 hours. The reaction solution was concentrated under reduced pressure, and the residue was purified by silica gel column chromatography. The obtained crude crystals were recrystallized from ethyl acetate to give Compound 2 (0.62 g).

$^1$H-NMR (270 MHz, DMSO-$d_6$) δ 3.91 (s, 6H), 7.19-7.26 (m, 2H), 7.40 (s, 2H), 7.48 (m, 1H), 7.65 (d, J = 15.6 Hz, 1H), 8.02 (d, J = 15.6 Hz, 1H), 8.05 (m, 1H), 8.11 (d, J = 2.5 Hz, 1H), 9.26 (s, 1H), 11.91 (s, 1H)
EI-MS m/z = 323 ($M^+$)

| Elemental analysis: $C_{19}H_{17}NO_4$ | | | |
|---|---|---|---|
| Calcd.(%): | C, 70.57; | H, 5.30; | N, 4.33 |
| Found (%): | C, 70.56; | H, 5.34; | N, 4.38 |

## Example 3

(E)-1-(4-Benzyloxy-3,5-dimethoxyphenyl)-3-(indol-3-yl)-2-propen-1-one (Compound 3)

4'-Hydroxy-3',5'-dimethoxyacetophenone (1.57 g) and benzyl bromide (1.35 g) were dissolved in acetone (50 ml), and potassium carbonate (1.70 g) was added thereto, followed by heating under reflux for 24 hours. Insoluble matters were filtered off, and the filtrate was concentrated under reduced pressure. The residue was washed with hexane and collected by filtration. The obtained crystals (1.96 g) and indole-3-carboxaldehyde (0.99 g) were dissolved in ethanol (10 ml), and piperidine (0.85 g) was added thereto, followed by heating under reflux for 32 hours. The reaction solution was concentrated under reduced pressure, and the residue was purified by silica gel column chromatography. The obtained crude crystals were recrystallized from a mixed solvent of ethyl acetate and hexane to give Compound 3 (0.17 g).

$^1$H-NMR (270 MHz, DMSO-$d_6$) δ 3.92 (s, 6H), 5.03 (s, 2H), 7.21-7.25 (m, 2H), 7.31-7.41 (m, 5H), 7.45-7.52 (m, 3H), 7.63 (d, J = 15.3 Hz, 1H), 8.05 (m, 1H), 8.06 (d, J = 15.3 Hz, 1H), 8.15 (d, J = 3.0Hz, 1H), 11.91 (s, 1H)
EI-MS m/z = 413 ($M^+$)

| Elemental analysis: $C_{26}H_{23}NO_4$ | | | |
|---|---|---|---|
| Calcd.(%): | C, 75.53; | H, 5.61; | N, 3.39 |
| Found (%): | C, 75.41; | H, 5.45; | N, 3.38 |

Example 4

3-(Indol-3-yl)-2-methyl-1-(3,4,5-triethoxyphenyl)-2-propen-1-one (Compound 4)

Process 1

3,4,5-Triethoxybenzoic acid (2.54 g) was dissolved in tetrahydrofuran (250 ml), and lithium aluminum hydride (1.20 g) was added thereto, followed by heating under reflux for 24 hours. Ethyl acetate and then a 2N aqueous solution of sodium hydroxide were added to the reaction solution, insoluble matters were filtered off, and the filtrate was concentrated under reduced pressure. The residue was dissolved in ethyl acetate (100 ml), and manganese dioxide (5.10 g) was added thereto, followed by stirring for 120 hours. Insoluble matters were filtered off, and the filtrate was concentrated under reduced pressure. The residue was dissolved in tetrahydrofuran (100 ml), and ethylmagnesium bromide (1M tetrahydrofuran solution, 15 ml) was added thereto, followed by stirring at room temperature for 30 minutes. 1N Hydrochloric acid was added to the reaction solution and the mixture was extracted with ethyl acetate. The organic layer was washed with a saturated saline, dried over anhydrous magnesium sulfate and concentrated under reduced pressure. The residue was dissolved in acetone (100 ml), and Jones' reagent (2 ml) was added thereto under ice-cooling, followed by stirring at the same temperature for 30 minutes. 2-Propanol (10 ml) was added to the reaction solution and the mixture was concentrated under reduced pressure. The residue was subjected to partitioning between ethyl acetate and water. The organic layer was washed with a saturated saline, dried over anhydrous magnesium sulfate and concentrated under reduced pressure. The residue was purified by silica gel column chromatography to give 3',4',5'-triethoxypropiophenone (0.76 g).

$^1$H-NMR (270 MHz, $CDCl_3$) $\delta$ 1.21 (t, J = 7.2 Hz, 3H), 1.37 (t, J = 6.9 Hz, 3H), 1.45 (t, J = 6.9 Hz, 6H), 2.95 (q, J = 7.2 Hz, 2H), 4.12(q, J = 6.9 Hz,4H), 4.14 (q, J = 6.9 Hz, 2H), 7.21 (s, 2H)
EI-MS m/z = 266 ($M^+$)

Process 2

3',4',5'-Triethoxypropiophenone (0.67 g) obtained in the above Process 1 and indole-3-carboxaldehyde (0.37 g) were dissolved in ethanol (5 ml), and piperidine (0.43 g) was added thereto, followed by heating under reflux for 32 hours. The reaction solution was concentrated under reduced pressure, and the residue was purified by silica gel column chromatography. The obtained crude crystals were recrystallized from ethanol to give Compound 4 (0.25 g).

$^1$H-NMR (270 MHz, $CDCl_3$) $\delta$ 1.41 (t, J = 7.0 Hz, 3H), 1.42 (t, J = 7.0 Hz, 6H), 2.31 (d, J = 1.0 Hz, 3H), 4.10 (q, J = 7.0 Hz, 4H), 4.18 (q, J = 7.0 Hz, 2H), 7.00 (s, 2H), 7.18 (m, 1H), 7.28 (m, 1H), 7.44 (d, J = 7.8 Hz, 1H), 7.57 (d, J = 7.9 Hz, 1H), 7.63 (d, J = 2.6 Hz, 1H), 7.66 (brs, 1H), 8.71 (brs, 1H)
EI-MS m/z = 393 ($M^+$)

| Elemental analysis: $C_{24}H_{27}NO_4$ | | | |
|---|---|---|---|
| Calcd.(%): | C, 73.26; | H, 6.92; | N, 3.56 |
| Found (%): | C, 73.43; | H, 7.32; | N, 3.54 |

Example 5

1-(4-Ethoxy-3,5-dimethoxyphenyl)-3-(indol-3-yl)-2-methyl-2-propen-1-one (Compound 5)

Process 1

4-Hydroxy-3,5-dimethoxybenzaldehyde (3.64 g) and ethyl iodide (6.24 g) were dissolved in N,N-dimethylformamide (30 ml), and sodium hydride (60% oil dispersion, 1.00 g) was added thereto, followed by stirring at 80°C for 24 hours. The reaction solution was subjected to partitioning between ethyl acetate and 1N hydrochloric acid. The organic layer was washed successively with a 5% aqueous solution of sodium bicarbonate, water and a saturated saline, dried over anhydrous magnesium sulfate and concentrated under reduced pressure. The residue (3.71 g) was dissolved in tetrahydrofuran (88 ml), and ethylmagnesium bromide (1M tetrahydrofuran solution, 26.5 ml) was added thereto, followed by stirring at room temperature for 30 minutes. 1N Hydrochloric acid was added to the reaction solution and the mixture was extracted with ethyl acetate. The organic layer was washed with a saturated saline, dried over anhydrous magnesium sulfate and concentrated under reduced pressure. The residue was dissolved in acetone (100 ml), and Jones' reagent (5.3 ml) was added thereto under ice-cooling, followed by stirring at the same temperature for 30 minutes. 2-Propanol (5.3 ml) was added to the reaction solution and the mixture was concentrated under reduced pressure. The residue was subjected to partitioning between ethyl acetate and water. The organic layer was washed with a saturated saline, dried over anhydrous magnesium sulfate and concentrated under reduced pressure. The residue was purified by silica gel column chromatography to give 4'-ethoxy-3',5'-dimethoxypropiophenone (3.37 g).

$^1$H-NMR (90 MHz, $CDCl_3$) $\delta$ 1.23 (t, J = 7.3 Hz, 3H), 1.34 (t, J = 7.0 Hz, 3H), 2.97 (q, J = 7.3 Hz, 2H), 3.90 (s, 6H), 4.13 (q, J = 7.0 Hz, 2H), 7.22 (s, 2H)
EI-MS m/z = 238 ($M^+$)

Process 2

4'-Ethoxy-3',5'-dimethoxypropiophenone (1.67 g) obtained in the above Process 1 and indole-3-carboxaldehyde (1.02 g) were dissolved in ethanol (14 ml), and piperidine (0.60 g) was added thereto, followed by heating under reflux for 144 hours. The reaction solution was concentrated under reduced pressure, and the residue was purified by silica gel column chromatography. The obtained crude crystals were recrystallized from ethyl acetate to give Compound 5 (1.20 g).

$^1$H-NMR (270 MHz, $CDCl_3$) $\delta$ 1.41 (t, J = 7.1 Hz, 3H), 2.31 (d, J = 1.0 Hz, 3H), 3.87 (s, 6H), 4.16 (q, J = 7.1 Hz, 2H), 7.01 (s, 2H), 7.18 (m, 1H), 7.27 (m, 1H), 7.43 (brd, J = 7.9 Hz, 1H), 7.56 (brd, J = 7.6 Hz, 1H), 7.63 (d, J = 3.0 Hz, 1H), 7.68 (brs, 1H), 8.75 (brs, 1H)
EI-MS m/z = 365 ($M^+$)

| Elemental analysis: $C_{22}H_{23}NO_4$ | | | |
|---|---|---|---|
| Calcd.(%): | C, 72.31; | H, 6.34; | N, 3.83 |
| Found (%): | C, 72.44; | H, 6.41; | N, 3.75 |

Example 6

3-(Indol-3-yl)-1-(4-isobutyloxy-3,5-dimethoxyphenyl)-2-methyl-2-propen-1-one (Compound 6)

Process 1

Substantially the same procedure as in Process 1 of Example 5 was repeated using 4-hydroxy-3,5-dimethoxybenzaldehyde (3.64 g) and isobutyl bromide (5.48 g) to give 4'-isobutyloxy-3',5'-dimethoxypropiophenone (1.92 g).

$^1$H-NMR (90 MHz, $CDCl_3$) $\delta$ 1.02 (d, J = 6.8 Hz, 6H), 1.22 (t, J = 7.2 Hz, 3H), 2.06 (m, 1H), 2.96 (q, J = 7.2 Hz, 2H),

3.81 (d, J = 6.6 Hz, 2H), 3.89 (s, 6H), 7.22 (s, 2H)
EI-MS m/z = 266 ($M^+$)

Process 2

4'-Isobutyloxy-3',5'-dimethoxypropiophenone (1.33 g) obtained in the above Process 1 and indole-3-carboxaldehyde (0.73 g) were dissolved in ethanol (10 ml), and piperidine (0.85 g) was added thereto, followed by heating under reflux for 48 hours. The reaction solution was concentrated under reduced pressure, and the residue was purified by silica gel column chromatography. The obtained crude crystals were recrystallized from a mixed solvent of ethyl acetate and hexane to give Compound 6 (0.90 g).

$^1$H-NMR (270 MHz, $CDCl_3$) δ 1.06 (d, J = 6.7 Hz, 6H), 2.11 (m, 1H), 2.31 (d, J = 1.0 Hz, 3H), 3.85 (d, J = 6.7 Hz, 2H), 3.87 (s, 6H), 7.02 (s, 2H), 7.19 (m, 1H), 7.28 (m, 1H), 7.44 (brd, J = 8.2 Hz, 1H), 7.57 (brd, J = 7.9 Hz, 1H), 7.64 (d, J = 2.6 Hz, 1H), 7.68 (brs, 1H), 8.73 (brs, 1H)
EI-MS m/z = 393 ($M^+$)

| Elemental analysis: $C_{24}H_{27}NO_4$ | | | |
|---|---|---|---|
| Calcd.(%): | C, 73.26; | H, 6.92; | N, 3.56 |
| Found (%): | C, 73.20; | H, 7.31; | N, 3.53 |

Example 7

1-(4-Ethyl-3-dimethoxyphenyl)-3-(indol-3-yl)-2-methyl-2-propen-1-one (Compound 7)

Process 1

3,4,5-Trimethoxybenznitrile (3.86 g) was dissolved in tetrahydrofuran (100 ml), and ethylmagnesium bromide (1M tetrahydrofuran solution, 60 ml) was added thereto, followed by heating under reflux for 12 hours. 1N Hydrochloric acid was added to the reaction solution, and the mixture was stirred at the same temperature for one hour and extracted with ethyl acetate. The organic layer was washed with a saturated saline, dried over anhydrous magnesium sulfate and concentrated under reduced pressure. The residue was purified by silica gel column chromatography to give 4'-ethyl-3',5'-dimethoxypropiophenone (2.37 g).

$^1$H-NMR (90 MHz, $CDCl_3$) δ 1.08 (t, J = 7.3 Hz, 3H), 1.23 (t, J = 7.3 Hz, 3H), 2.69 (q, J = 7.3 Hz, 2H), 2.97 (q, J = 7.3 Hz, 2H), 3.87(s, 6H), 7.15 (s, 2H)
EI-MS m/z = 222 ($M^+$)

Process 2

4'-Ethyl-3',5'-dimethoxypropiophenone (1.11 g) obtained in the above Process 1 and indole-3-carboxaldehyde (1.45 g) were dissolved in ethanol (10 ml), and piperidine (0.85 g) was added thereto, followed by heating under reflux for 27 hours. The reaction solution was concentrated under reduced pressure, and the residue was purified by silica gel column chromatography. The obtained crude crystals were recrystallized from a mixed solvent of ethyl acetate and 2-propanol and then from a mixed solvent of ethyl acetate and hexane to give Compound 7 (0.45 g).

$^1$H-NMR (270 MHz, $CDCl_3$) δ 1.14 (t, J = 7.4 Hz, 3H), 2.32 (d, J = 1.0 Hz, 3H), 2.74 (q, J = 7.4 Hz, 2H), 3.85 (s, 6H), 6.96 (s, 2H), 7.19 (m, 1H), 7.28 (m, 1H), 7.44 (brd, J = 8.4 Hz, 1H), 7.59 (brd, J = 7.9 Hz, 1H), 7.63 (d, J = 2.5 Hz, 1H), 7.72 (brs, 1H), 8.68 (brs, 1H)
EI-MS m/z = 365 ($M^+$)

| Elemental analysis: $C_{22}H_{23}NO_3$ | | | |
|---|---|---|---|
| Calcd.(%): | C, 75.62; | H, 6.63; | N, 4.01 |
| Found (%): | C, 75.78; | H, 6.78; | N, 3.98 |

Example 8

3-(Indol-3-yl)-1-(3-methoxy-4,5-methylenedioxyphenyl)-2-methyl-2-propen-1-one (Compound 8)

Process 1

3-Methoxy-4,5-methylenedioxybenzaldehyde (5.55 g) was dissolved in tetrahydrofuran (150 ml), and ethylmagnesium bromide (1M tetrahydrofuran solution, 46.2 ml) was added thereto, followed by stirring at room temperature for 30 minutes. 1N Hydrochloric acid was added to the reaction solution, and the mixture was extracted with ethyl acetate. The organic layer was washed successively with water, a 5% aqueous solution of sodium bicarbonate and a saturated saline, dried over anhydrous magnesium sulfate and concentrated under reduced pressure. The residue was dissolved in acetone (300 ml), and Jones' reagent (5.3 ml) was added thereto under ice-cooling, followed by stirring at the same temperature for 30 minutes. 2-Propanol (5.3 ml) was added to the reaction solution and the mixture was concentrated under reduced pressure. The residue was subjected to partitioning between ethyl acetate and water. The organic layer was washed successively with water and a saturated saline, dried over anhydrous magnesium sulfate and concentrated under reduced pressure. The residue was purified by silica gel column chromatography to give 3'-methoxy-4',5'-methylenedioxypropiophenone (5.27 g).

$^1$H-NMR (90 MHz, $CDCl_3$) $\delta$ 1.21 (t, J = 7.3 Hz, 3H), 2.91 (q, J = 7.3 Hz, 2H), 3.94 (s, 3H), 6.05 (s, 2H), 7.13 (d, J = 1.5 Hz, 1H), 7.27 (d, J = 1.5 Hz, 1H)
EI-MS m/z = 208 ($M^+$)

Process 2

3'-Methoxy-4',5'-methylenedioxypropiophenone (1.46 g) obtained in the above Process 1 and indole-3-carboxaldehyde (1.02 g) were dissolved in ethanol (14 ml), and piperidine (0.69 g) was added thereto, followed by heating under reflux for 120 hours. The precipitated crystals were collected by filtration, and the obtained crude crystals were recrystallized from a mixed solvent of ethyl acetate and hexane to give Compound 8 (0.64 g).

$^1$H-NMR (270 MHz, $CDCl_3$) $\delta$ 2.29 (s, 3H), 3.92 (s, 3H), 6.08 (s, 2H), 6.98 (d, J = 1.5 Hz, 1H), 7.06 (d, J = 1.5 Hz, 1H), 7.19 (m, 1H), 7.27 (m, 1H), 7.43 (brd, J = 7.9 Hz, 1H), 7.58-7.61 (m 3H), 8.68 (brs, 1H)
EI-MS m/z = 335 ($M^+$)

| Elemental analysis: $C_{20}H_{17}NO_4$ | | | |
|---|---|---|---|
| Calcd.(%): | C, 71.63; | H, 5.11; | N, 4.18 |
| Found (%): | C, 71.31; | H, 5.14; | N, 4.06 |

Example 9

1-(5-Ethoxy-3,4-dimethoxyphenyl)-3-(indol-3-yl)-2-methyl-2-propen-1-one (Compound 9)

Process 1

Substantially the same procedure as in Process 1 of Example 5 was repeated using 5-hydroxy-3,4-dimethoxybenzaldehyde (3.34 g) and ethyl iodide (5.72 g) to give 5'-ethoxy-3',4'-dimethoxypropiophenone (1.92 g).

$^1$H-NMR (90 MHz, $CDCl_3$) δ 1.22 (t, J = 7.3 Hz, 3H), 1.46 (t, J = 7.0 Hz, 3H), 2.96 (q, J = 7.3 Hz, 2H), 3.91 (s, 6H), 4.14 (q, J = 7.0 Hz, 2H), 7.22 (s, 2H)
EI-MS m/z = 238 ($M^+$)

Process 2

5'-Ethoxy-3',4'-dimethoxypropiophenone (1.19 g) obtained in the above Process 1 and indole-3-carboxaldehyde (0.73 g) were dissolved in ethanol (10 ml), and piperidine (0.43 g) was added thereto, followed by heating under reflux for 72 hours. The precipitated crystals were collected by filtration, and the obtained crude crystals were recrystallized from a mixed solvent of acetone and hexane to give Compound 9 (0.75 g).

$^1$H-NMR (270 MHz, $CDCl_3$) δ 1.44 (t, J = 7.1 Hz, 3H), 2.32 (d, J = 1.0 Hz, 3H), 3.89 (s, 3H), 3.96 (s, 3H), 4.12 (q, J = 7.1 Hz, 2H), 7.01 (s, 2H), 7.19 (m, 1H), 7.28 (m, 1H), 7.44 (brd, J = 7.9 Hz, 1H), 7.58 (brd, J = 7.9 Hz, 1H), 7.64 (d, J = 3.0 Hz, 1H), 7.67 (brs, 1H), 8.76 (brs, 1H)
EI-MS m/z = 365 ($M^+$)

| Elemental analysis: $C_{22}H_{23}NO_4$ | | | |
|---|---|---|---|
| Calcd.(%): | C, 72.31; | H, 6.34; | N, 3.83 |
| Found (%): | C, 72.36; | H, 6.62; | N, 3.80 |

Example 10

1-(3-Bromo-4-dimethoxyphenyl)-3-(indol-3-yl)-2-methyl-2-propen-1-one (Compound 10)

Process 1

Substantially the same procedure as in Process 1 of Example 5 was repeated using 5-bromovanillin (5.78 g) and methyl iodide (7.10 g) to give 3'-bromo-4',5'-dimethoxypropiophenone (5.45 g).

$^1$H-NMR (90 MHz, $CDCl_3$) δ 1.21 (t, J = 7.3 Hz, 3H), 2.94 (q, J = 7.3 Hz, 2H), 3.92 (s, 6H), 7.49 (d, J = 1.9 Hz, 1H), 7.74 (d, J = 1.9 Hz, 1H)
EI-MS m/z = 272, 274 ($M^+$)

Process 2

3'-Bromo-4',5'-dimethoxypropiophenone (1.37 g) obtained in the above Process 1 and indole-3-carboxaldehyde (0.73 g) were dissolved in ethanol (10 ml), and piperidine (0.43 g) was added thereto, followed by heating under reflux for 72 hours. The reaction solution was concentrated under reduced pressure, and the residue was purified by silica gel column chromatography. The obtained crude crystals were recrystallised from ethyl acetate to give Compound 10 (0.49 g).

$^1$H-NMR (270 MHz, $CDCl_3$) δ 2.30 (d, J = 0.7 Hz, 3H), 3.92 (s, 3H), 3.96 (s, 3H), 7.21 (m, 1H), 7.23 (d, J = 2.1 Hz, 1H), 7.29 (m, 1H), 7.44 (brd, J = 7.8 Hz, 1H), 7.55 (d, J = 2.1 Hz, 1H), 7.61 (brd, J = 7.8 Hz, 1H), 7.64 (d, J = 2.1

Hz, 1H), 7.65 (brs, 1H), 8.71 (brs, 1H)
EI-MS m/z = 399, 401 ($M^+$)

| Elemental analysis: $C_{20}H_{18}BrNO_3$ | | | |
|---|---|---|---|
| Calcd.(%): | C, 60.01; | H, 4.53; | N, 3.50 |
| Found (%): | C, 60.18; | H, 4.61; | N, 3.40 |

Example 11

1-(4-Ethoxy-3,5-dimethoxyphenyl)-2-methyl-3-(6-methylindol-3-yl)-2-propen-1-one (Compound 11)

4'-Ethoxy-3',5'-dimethoxypropiophenone (1.19 g) obtained in Process 1 of Example 5 and 6-methylindole-3-carboxaldehyde [Journal of the Organic Chemistry (J. Org. Chem.), 44, 3741 (1979)] (0.80 g) were dissolved in ethanol (10 ml), and piperidine (0.49 g) was added thereto, followed by heating under reflux for 48 hours. The reaction solution was concentrated under reduced pressure, and the residue was purified by silica gel column chromatography. The obtained crude crystals were recrystallized from ethyl acetate to give Compound 11 (1.20 g).

$^1$H-NMR (270 MHz, $CDCl_3$) δ 1.41 (t, J = 7.1 Hz, 3H), 2.30 (d, J = 1.3 Hz, 3H), 2.47 (s, 3H), 3.87 (s, 6H), 4.17 (q, J = 7.1 Hz, 2H), 7.01 (dd, J = 7.6, 0.7 Hz, 1H), 7.02 (s, 2H), 7.22 (s, 1H), 7.44 (d, J = 7.6 Hz, 1H), 7.56 (d, J = 2.6 Hz, 1H), 7.66 (s, 1H), 8.53 (brs, 1H)
FAB-MS m/z = 380 ($M^+$ + 1)

| Elemental analysis: $C_{23}H_{25}NO_4$ | | | |
|---|---|---|---|
| Calcd.(%): | C, 72.80; | H, 6.64; | N, 3.69 |
| Found (%): | C, 72.82; | H, 6.78; | N, 3.69 |

Example 12

1-(3-Methoxy-4,5-methylenedioxyphenyl)-2-methyl-3-(6-methylindol-3-yl)-2-propen-1-one (Compound 12)

3'-Methoxy-4',5'-methylenedioxypropiophenone (1.04 g) obtained in Process 1 of Example 8 and 6-methylindole-3-carboxaldehyde [Journal of the Organic Chemistry (J. Org. Chem.), 44, 3741 (1979)] (0.80 g) were dissolved in ethanol (10 ml), and piperidine (0.49 g) was added thereto, followed by heating under reflux for 48 hours. The precipitated crystals were collected by filtration, and the obtained crude crystals were recrystallised from a mixed solvent of ethyl acetate and hexane to give Compound 12 (0.64 g).

$^1$H-NMR (270 MHz, $CDCl_3$) δ 2.29 (d, J = 1.0 Hz, 3H), 2.24 (s, 3H), 3.93 (s, 3H), 6.08 (s, 2H), 6.98 (d, J = 1.3 Hz, 1H), 7.02 (d, J = 7.6 Hz, 1H), 7.06 (d, J = 1.3 Hz, 1H), 7.22 (s, 1H), 7.47 (d, J = 7.6 Hz, 1H), 7.54 (d, J = 2.6 Hz, 1H), 7.59 (brs, 1H), 8.51 (brs, 1H)
FAB-MS m/z = 350 ($M^+$ + 1)

| Elemental analysis: $C_{21}H_{19}NO_4$ | | | |
|---|---|---|---|
| Calcd.(%): | C, 72.19; | H, 5.48; | N, 4.01 |
| Found (%): | C, 72.41; | H, 5.47; | N, 3.99 |

Example 13

1-(3-Bromo-4-isobutyloxy-5-methoxyphenyl)-3-(indol-3-yl)-2-methyl-2-propen-1-one (Compound 13)

Process 1

Substantially the same procedure as in Process 1 of Example 5 was repeated using 5-bromovanillin (11.65 g) and isobutyl bromide (27.40 g) to give 3'-bromo-4'-isobutyloxy-5'-methoxypropiophenone (1.93 g).

$^1$H-NMR (270 MHz, $CDCl_3$) δ 1.06 (d, J = 6.6 Hz, 6H), 1.21 (t, J = 7.0 Hz, 3H), 2.13 (m, 1H), 2.94 (q, J = 7.0 Hz, 2H), 3.84 (d, J = 6.6 Hz, 2H), 3.89 (s, 3H), 7.48 (d, J = 1.9 Hz, 1H), 7.74 (d, J = 1.9 Hz, 1H)
EI-MS m/z = 314, 316 ($M^+$)

Process 2

3'-Bromo-4'-isobutyloxy-5'-methoxypropiophenone (0.64 g) obtained in the above Process 1 and indole-3-carboxaldehyde (0.59 g) were dissolved in ethanol (1 ml), and piperidine (0.35 g) was added thereto, followed by heating under reflux for 25 hours. The reaction solution was concentrated under reduced pressure, and the residue was purified by silica gel column chromatography. The obtained crude crystals were recrystallized from a mixed solvent of ethyl acetate and hexane to give Compound 13 (0.54 g).

$^1$H-NMR (270 MHz, $CDCl_3$) δ 1.10 (d, J = 6.4 Hz, 6H), 2.17 (m, 1H), 2.30 (d, J = 0.7 Hz, 3H), 3.88 (d, J = 6.4 Hz, 2H), 3.89 (s, 3H), 7.22 (m, 1H), 7.27 (d, J = 1.7 Hz, 1H), 7.30 (m, 1H), 7.44 (m, 1H), 7.55 (d, J = 1.7 Hz, 1H), 7.58 (d, J = 8.1 Hz, 1H), 7.63 (d, J = 2.0 Hz, 1H), 7.63 (s, 1H), 8.67 (s, 1H)
EI-MS m/z = 441, 443 ($M^+$)

| Elemental analysis: $C_{23}H_{24}BrNO_3$ | | | |
|---|---|---|---|
| Calcd.(%): | C, 62.45; | H, 5.47; | N, 3.17 |
| Found (%): | C, 62.65; | H, 5.49; | N, 3.15 |

Example 14

1-(3-Bromo-4-isobutyloxy-5-methoxyphenyl)-2-methyl-3-(6-methylindol-3-yl)-2-propen-1-one (Compound 14)

3'-Bromo-4'-isobutyloxy-5'-methoxypropiophenone (1.58 g) obtained in Process 1 of Example 13 and 6-methylindole-3-carboxaldehyde [Journal of the Organic Chemistry (J. Org. Chem.), 44, 3741 (1979)] (0.80 g) were dissolved in ethanol (2 ml), and piperidine (0.85 g) was added thereto, followed by heating under reflux for 48 hours. The reaction solution was concentrated under reduced pressure, and the residue was purified by silica gel column chromatography. The obtained crude crystals were recrystallized from a mixed solvent of ethyl acetate and hexane to give Compound 14 (1.02 g).

$^1$H-NMR (270 MHz, $CDCl_3$) δ 1.10 (d, J = 6.3 Hz, 6H), 2.17 (m, 1H), 2.29 (d, J = 1.0 Hz, 3H), 2.47 (s, 3H), 3.87 (d,

J = 6.3 Hz, 2H), 3.88 (s, 3H), 7.04 (dd, J = 8.3, 1.0 Hz, 1H), 7.22 (s, 1H), 7.26 (d, J = 1.7 Hz, 1H), 7.45 (d, J = 8.3 Hz, 1H), 7.54 (d, J = 1.7 Hz, 1H), 7.56(d, J = 3.0 Hz, 1H), 7.62 (s, 1H), 8.57 (brs, 1H)
EI-MS m/z = 455, 457 ($M^+$)

| Elemental analysis: $C_{24}H_{26}BrNO_3$ | | | |
|---|---|---|---|
| Calcd.(%): | C, 63.28; | H, 5.76; | N, 3.08 |
| Found (%): | C, 63.66; | H, 6.11; | N, 2.88 |

Example 15

1-(4-Isobutyloxy-3,5-dimethoxyphenyl)-2-methyl-3-(6-methylindol-3-yl)-2-methyl-2-propen-1-one (Compound 15)

Process 1

4-Hydroxy-3,5-dimethoxybenzaldehyde (36.40 g) was dissolved in a mixed solvent of tetrahydrofuran (250 ml) and N,N-dimethylformamide (250 ml), and isobutyl bromide (54.80 g) and tetra-n-butylammonium fluoride (1M tetrahydrofuran solution, 400 ml) were added thereto, followed by heating under reflux for 15 hours. The reaction solution was cooled to room temperature, and subjected to partitioning between hexane and water. The organic layer was washed with water, dried over anhydrous magnesium sulfate and concentrated under reduced pressure to give 4'-isobutyloxy-3',5'-dimethoxybenzaldehyde (41.17 g).

$^1$H-NMR (90 MHz, $CDCl_3$) $\delta$ 1.30 (d, J = 6.6 Hz, 6H), 2.04 (m, 1H), 3.84 (d, J = 6.6 Hz, 2H), 3.91 (s, 6H), 7.12 (s, 2H), 9.86 (s, 1H)
EI-MS m/z = 238 ($M^+$)

Process 2

Substantially the same procedure as in Process 1 of Example 8 was repeated using 4'-isobutyloxy-3',5'-dimethoxybenzaldehyde (1.37 g) obtained in the above Process 1 to give 4'-isobutyloxy-3',5'-dimethoxypropiophenone (1.37 g).

Process 3

4'-Isobutyloxy-3',5'-dimethoxypropiophenone (1.33 g) obtained in the above Process 2 and 6-methylindole-3-carboxaldehyde [Journal of the Organic Chemistry (J. Org. Chem.), 44, 3741 (1979)] (0.80 g) were dissolved in ethanol (2 ml), and piperidine (0.43 g) was added thereto, followed by heating under reflux for 80 hours. The reaction solution was concentrated under reduced pressure, and the residue was purified by silica gel column chromatography. The obtained crude crystals were recrystallised from a mixed solvent of ethyl acetate and hexane to give Compound 15 (0.96 g).

$^1$H-NMR (270 MHz, $CDCl_3$) $\delta$ 1.00 (d, J = 7.8 Hz, 6H), 2.05 (m, 1H), 2.25 (s, 3H), 2.41 (s, 3H), 3.79 (d, J = 7.8 Hz, 2H), 3.81 (s, 6H), 6.956 (d, J = 7.8 Hz, 1H), 6.957 (s, 2H), 7.16 (s, 1H), 7.38 (d, J = 7.8 Hz, 1H), 7.50 (d, J = 2.6 Hz, 1H), 7.60 (s, 1H), 8.53 (s, 1H)
EI-MS m/z = 407 ($M^+$)

| Elemental analysis: $C_{25}H_{29}NO_4$ | | | |
|---|---|---|---|
| Calcd.(%): | C, 73.69; | H, 7.17; | N, 3.44 |
| Found (%): | C, 74.13; | H, 7.23; | N, 3.40 |

Example 16

1-(4-Ethoxy-3,5-dimethoxyphenyl)-3-(6-ethylindol-3-yl)-2-methyl-2-propen-1-one (Compound 16)

Process 1

Phosphorus oxychloride (3.82 g) was added to N,N-dimethylformamide (20 ml), and the mixture was stirred at room temperature for 10 minutes. A solution of 6-ethylindole [Journal of the Chemical Society (J. Chem. Soc.), 7165 (1965)] in N,N-dimethylformamide (6 ml) was added to the reaction solution, and the mixture was stirred at room temperature for one hour. Ice (20 g) and then a 5N aqueous solution of sodium hydroxide (34 ml) were added to the reaction solution, and the mixture was heated under reflux for one hour. The reaction solution was ice-cooled, and the precipitated crystals were collected by filtration to give 6-ethylindole-3-carboxaldehyde (2.76 g).

$^1$H-NMR (90 MHz, $CDCl_3$) $\delta$ 1.27 (t, J = 7.6 Hz, 3H), 2.76 (q, J = 7.6 Hz, 2H), 7.11-7.24 (m, 2H), 7.76 (s, 1H), 8.20 (d, J = 8.2 Hz, 1H), 8.99 (s, 1H), 10.01 (s, 1H)
EI-MS m/z = 173 ($M^+$)

Process 2

Substantially the same procedure as in Process 2 of Example 6 was repeated using 4'-ethoxy-3',5'-dimethoxypropiophenone (1.90 g) obtained in Process 1 of Example 5 and 6-ethylindole-3-carboxaldehyde (1.38 g) to give Compound 16 (0.84 g).

$^1$H-NMR (270 MHz, $CDCl_3$) $\delta$ 1.30 (t, J = 7.4 Hz, 3H), 1.41 (t, J = 7.4 Hz, 3H), 2.30 (d, J = 1.0 Hz, 3H), 2.76 (q, J = 7.4 Hz, 2H), 3.87(s, 6H), 4.17 (q, J = 7.4 Hz, 2H), 7.02 (s, 2H), 7.05 (dd, J = 8.2, 1.5 Hz, 1H), 7.24 (s, 1H), 7.46 (d, J = 8.2 Hz, 1H), 7.57 (d, J = 2.5 Hz, 1H), 7.67 (s, 1H), 8.61 (s, 1H)
EI-MS m/z = 393 ($M^+$)

| Elemental analysis: $C_{24}H_{27}NO_4$ | | | |
|---|---|---|---|
| Calcd.(%): | C, 73.26; | H, 6.92; | N, 3.56 |
| Found (%): | C, 73.46; | H, 7.02; | N, 3.52 |

Example 17

1-(4-Ethoxy-3,5-dimethoxyphenyl)-3-(6-isopropylindol-3-yl)-2-methyl-2-propen-1-one (Compound 17)

Process 1

Substantially the same procedure as in Process 1 of Example 16 was repeated using 6-isopropylindole [Organic Synthesis (Org. Syn.), 63, 214 (1985)] (3.18 g) to give 6-isopropylindole-3-carboxaldehyde (3.56 g).

$^1$N-NMR (90 MHz, $CDCl_3$) $\delta$ 1.31 (d, J = 8.7 Hz, 6H), 2.70 (m, 1H), 7.08 (m, 1H), 7.24 (s, 1H), 7.74 (brs, 1H), 8.18 (d, J = 9.0 Hz, 1H), 9.28 (s, 1H), 9.97 (s, 1H)
EI-MS m/z = 187 ($M^+$)

Process 2

Substantially the same procedure as in Process 2 of Example 6 was repeated using 4'-ethoxy-3',5'-dimethoxypropiophenone (2.38 g) obtained in Process 1 of Example 5 and 6-isopropylindole-3-carboxaldehyde obtained in the above Process 1 (1.87 g) to give Compound 17 (1.80 g).

$^{1}$H-NMR (270 MHz, $CDCl_3$) δ 1.30 (d, J = 6.9 Hz, 6H), 1.41 (t, J = 7.0 Hz, 3H), 2.30 (d, J = 1.0 Hz, 3H), 3.03 (m, 1H), 3.88 (s, 6H), 4.17 (q, J = 7.0 Hz, 2H), 7.02 (s, 2H), 7.08 (dd, J = 8.4, 1.5 Hz, 1H), 7.28 (s, 1H), 7.47 (d, J = 8.4 Hz, 1H), 7.57 (d, J = 2.5 Hz, 1H), 7.66 (s, 1H), 8.60 (s, 1H)
EI-MS m/z = 407 ($M^+$)

| Elemental analysis: $C_{25}H_{29}NO_4$ | | | |
|---|---|---|---|
| Calcd.(%): | C, 73.69; | H, 7.17; | N, 3.44 |
| Found (%): | C, 73.85; | H, 7.27; | N, 3.43 |

Example 18

3-(6-Chloroindol-3-yl)-1-(4-ethoxy-3,5-dimethoxyphenyl)-2-methyl-2-propen-1-one (Compound 18)

Process 1

Substantially the same procedure as in Process 1 of Example 16 was repeated using 6-chloroindole (4.11 g) to give 6-chloroindole-3-carboxaldehyde (4.78 g).

$^{1}$H-NMR (270 MHz, DMSO-$d_6$) δ 7.24 (dd, J = 8.5, 1.8 Hz, 1H), 7.56 (d, J = 1.8 Hz, 1H), 8.07 (d, J = 8.5 Hz, 1H), 8.32 (s, 1H), 9.93 (s, 1H), 12.20 (s, 1H)
FAB-MS m/z = 180, 182 ($M^+$ + 1)

Process 2

Substantially the same procedure as in Process 2 of Example 6 was repeated using 4'-ethoxy-3',5'-dimethoxypropiophenone (2.38 g) obtained in Process 1 of Example 5 and 6-chloroindole-3-carboxaldehyde obtained in the above Process 1 (1.80 g) to give Compound 18 (1.69 g).

$^{1}$H-NMR (270 MHz, $CDCl_3$) δ 1.41 (t, J = 7.0 Hz, 3H), 2.31 (d, J = 0.9 Hz, 3H), 3.88 (s, 6H), 4.17 (q, J = 7.0 Hz, 2H), 7.01 (s, 2H), 7.15 (dd, J = 8.6, 1.8 Hz, 1H), 7.43 (d, J = 1.8 Hz, 1H), 7.46 (d, J = 8.6 Hz, 1H), 7.58 (s, 1H), 7.61 (d, J = 2.6 Hz, 1H), 8.72 (s, 1H)
EI-MS m/z = 399, 401 ($M^+$)

| Elemental analysis: $C_{22}H_{22}ClNO_4$ | | | |
|---|---|---|---|
| Calcd.(%): | C, 66.08; | H, 5.55; | N, 3.50 |
| Found (%): | C, 66.28; | H, 5.64; | N, 3.48 |

Example 19

1-(3,5-Dimethoxy-4-propoxyphenyl)-2-methyl-3-(6-methylindol-3-yl)-2-propen-1-one (Compound 19)

Process 1

Substantially the same procedure as in Process 1 of Example 5 was repeated using 4-hydroxy-3,5-dimethoxybenzaldehyde (9.10 g) and propyl iodide (12.75 g) to give 3',5'-dimethoxy-4'-propoxypropiophenone (4.85 g).

$^1$H-NMR (270 MHz, $CDCl_3$) δ 1.00 (t, J = 7.0 Hz, 3H), 1.21 (t, J = 7.3 Hz, 3H), 1.76 (m, 2H), 2.96 (q, J = 7.3 Hz, 2H), 3.88 (s, 6H), 4.00 (t, J = 7.0 Hz, 2H), 7.21 (s, 2H)
EI-MS m/z = 252 ($M^+$)

Process 2

Substantially the same procedure as in Process 2 of Example 6 was repeated using 3',5'-dimethoxy-4'-propoxypropiophenone (2.48 g) obtained in the above Process 1 and 6-methylindole-3-carboxaldehyde [Journal of the Organic Chemistry (J. Org. Chem.), 44, 3741 (1979)] (1.59 g) to give Compound 19 (1.73 g).

$^1$H-NMR (270 MHz, $CDCl_3$) δ 1.05 (t, J = 7.1 Hz, 3H), 1.83 (m, 2H), 2.31 (s, 3H), 2.48 (s, 3H), 3.88 (s, 6H), 4.05 (t, J = 7.1 Hz, 2H), 7.021 (s, 2H), 7.024 (m, 1H), 7.23 (s, 1H), 7.44 (d, J = 8.3 Hz, 1H), 7.57 (d, J = 2.6 Hz, 1H), 7.67 (s, 1H ), 8.59 (s, 1H)
EI-MS m/z = 393 ($M^+$)

| Elemental analysis: $C_{24}H_{27}NO_4$ | | | |
|---|---|---|---|
| Calcd.(%): | C, 73.26; | H, 6.92; | N, 3.56 |
| Found (%): | C, 73.44; | H, 7.02; | N, 3.51 |

Example 20

1-(4-Buthoxy-3,5-dimethoxyphenyl)-2-methyl-3-(6-methylindol-3-yl)-2-propen-1-one (Compound 20)

Process 1

Substantially the same procedure as in Process 1 of Example 5 was repeated using 4-hydroxy-3,5-dimethoxybenzaldehyde (9.10 g) and butyl bromide (10.28 g) to give 4'-butoxy-3',5'-dimethoxypropiophenone (5.42 g).

$^1$H-NMR (270 MHz, $CDCl_3$) δ 0.93 (t, J = 7.3 Hz, 3H), 1.20 (t, J = 7.3 Hz, 3H), 1.46 (m, 2H), 1.74 (m, 2H), 2.96 (q, J = 7.3 Hz, 2H), 3.90 (s, 6H), 4.02 (t, J = 7.3 Hz, 2H), 7.20 (s, 2H)
EI-MS m/z = 266 ($M^+$)

Process 2

Substantially the same procedure as in Process 2 of Example 6 was repeated using 4'-butoxy-3',5'-dimethoxypropiophenone (2.66 g) obtained in the above Process 1 and 6-methylindole-3-carboxaldehyde [Journal of the Organic Chemistry (J. Org. Chem.), 44, 3741 (1979)] (1.59 g) to give Compound 20 (1.43 g).

$^1$H-NMR (270 MHz, $CDCl_3$) δ 0.97 (t, J = 7.4 Hz, 3H), 1.53 (m, 2H), 1.81 (m, 2H), 2.29 (s, 3H), 2.45 (s, 3H), 3.85 (s, 6H), 4.07 (t, J = 6.8 Hz, 2H), 6.996 (s, 2H), 6.999 (d, J = 7.6 Hz, 1H), 7.20 (s, 1H), 7.41 (d, J = 7.6 Hz, 1H), 7.54 (d, J = 2.6 Hz, 1H), 7.64 (s, 1H), 8.55 (s, 1H)
EI-MS m/z = 407 ($M^+$)

| Elemental analysis: $C_{25}H_{29}NO_4$ | | | |
|---|---|---|---|
| Calcd.(%): | C, 73.69; | H, 7.17; | N, 3.44 |
| Found (%): | C, 73.85; | H, 7.29; | N, 3.46 |

Example 21

1-(2,5-Dimethoxyphenyl)-3-(indol-3-yl)-2-propen-1-one (Compound 21)

2',5'-Dimethoxyacetophenone (1.80 g) and indole-3-carboxaldehyde (1.45 g) were dissolved in ethanol (20 ml), and piperidine (0.85 g) was added thereto, followed by heating under reflux for 72 hours. The precipitated crystals were collected by filtration, and the obtained crude crystals were recrystallized from ethanol to give Compound 21 (1.35 g).

$^1$H-NMR (270 MHz, $CDCl_3$) δ 3.82 (s, 3H), 3.89 (s, 3H), 6.96 (d, J = 8.9 Hz, 1H), 7.04 (dd, J = 8.9, 3.0 Hz, 1H), 7.23 (d, J = 3.0 Hz, 1H), 7.28 (m, 2H), 7.43 (m, 1H), 7.51 (d, J = 15.8 Hz, 1H), 7.55 (d, J = 3.5 Hz, 1H), 7.92 (d, J = 15.8 Hz, 1H), 7.97 (m, 1H), 8.56(brs, 1H)
EI-MS m/z = 307 ($M^+$)

| Elemental analysis: $C_{19}H_{17}NO_3$ | | | |
|---|---|---|---|
| Calcd.(%): | C, 74.25; | H, 5.58; | N, 4.56 |
| Found (%): | C, 74.30; | H, 5.60; | N, 4.44 |

Example 22

1-(2,5-Dimethoxyphenyl)-3-(indol-3-yl)-2-methyl-2-propen-1-one (Compound 22)

Process 1

Substantially the same procedure as in Process 1 of Example 8 was repeated using 2,5-dimethoxybenzaldehyde (11.62 g) to give 2',5'-dimethoxypropiophenone (7.83 g).

$^1$H-NMR (90 MHz, $CDCl_3$) δ 1.15 (t, J = 7.3 Hz, 3H), 2.99 (q, J = 7.3 Hz, 2H), 3.78 (s, 3H), 3.84 (s, 3H), 6.82-7.08 (m, 2H), 7.23 (d, J = 2.9 Hz, 1H)
EI-MS m/z = 194 ($M^+$)

Process 2

2',5'-Dimethoxypropiophenone (1.94 g) obtained in the above Process 1 and indole-3-carboxaldehyde (1.45 g) were dissolved in ethanol (20 ml), and piperidine (0.85 g) was added thereto, followed by heating under reflux for 72 hours. The reaction solution was concentrated under reduced pressure, and the residue was purified by silica gel column chromatography. The obtained crude crystals were recrystallized from a mixed solvent of ethyl acetate and hexane to give Compound 22 (0.64 g).

$^1$H-NMR (270 MHz, $CDCl_3$) δ 2.28 (d, J = 1.0 Hz, 3H), 3.74 (s, 3H), 3.79 (s, 3H), 6.87 (d, J = 2.6 Hz, 1H), 6.93 (d, J = 8.8 Hz, 1H), 6.98 (dd, J = 8.8, 2.6 Hz, 1H), 7.15 (m, 1H), 7.24 (m, 1H), 7.37-7.46 (m, 2H), 7.607 (brs, 1H), 7.613 (d, J = 3.0 Hz, 1H), 8.76 (brs, 1H)
EI-MS m/z = 321 ($M^+$)

| Elemental analysis: $C_{20}H_{19}NO_3$ | | | |
|---|---|---|---|
| Calcd.(%): | C, 74.75; | H, 5.96; | N, 4.36 |
| Found (%): | C, 75.11; | H, 6.12; | N, 4.28 |

Example 23

3-(Indol-3-yl)-2-methyl-1-(2,4,5-trimethoxyphenyl)-2-propen-1-one (Compound 23)

Process 1

Substantially the same procedure as in Process 1 of Example 8 was repeated using 2,4,5-trimethoxybenzaldehyde (1.96 g) to give 2',4',5'-trimethoxypropiophenone (0.50 g).

$^1$H-NMR (270 MHz, $CDCl_3$) δ 1.16 (t, J = 7.3 Hz, 3H), 2.99 (q, J = 7.3 Hz, 2H), 3.88 (s, 3H), 3.91 (s, 3H), 3.95 (s, 3H), 6.50 (s, 1H), 7.43 (s, 1H)
FAB-MS m/z = 225 ($M^+$ + 1)

Process 2

2',4',5'-Trimethoxypropiophenone (0.45 g) obtained in the above Process 1 and indole-3-carboxaldehyde (0.28 g) were dissolved in ethanol (5 ml), and piperidine (0.17 g) was added thereto, followed by heating under reflux for 144 hours. The reaction solution was concentrated under reduced pressure, and the residue was purified by silica gel column chromatography. The obtained crude crystals were recrystallized from a mixed solvent of ethyl acetate and hexane to give Compound 23 (0.21 g).

$^1$H-NMR (270 MHz, $CDCl_3$) δ 2.28 (d, J = 0.7 Hz, 3H), 3.77 (s, 3H), 3.85 (s, 3H), 3.98 (s, 3H), 6.62 (s, 1H), 6.92 (s, 1H), 7.16 (m, 1H), 7.23 (d, J = 2.1 Hz, 1H), 7.42 (brd, J = 8.2 Hz, 1H), 7.50 (d, J = 7.9 Hz, 1H), 7.62-7.65 (m, 2H), 8.74 (brs, 1H)
EI-MS m/z = 351 ($M^+$)

| Elemental analysis: $C_{21}H_{21}NO4 \cdot 0.2H_2O$ | | | |
|---|---|---|---|
| Calcd.(%): | C, 71.05; | H, 6.08; | N, 3.95 |
| Found (%): | C, 71.05; | H, 6.13; | N, 3.83 |

Example 24

3-(Indol-3-yl)-2-methyl-1-(2,3,4-trimethoxyphenyl)-2-propen-1-one (Compound 24)

Process 1

Substantially the same procedure as in Process 1 of Example 8 was repeated using 2,3,4-trimethoxybenzaldehyde (1.96 g) to give 2',3',4'-trimethoxypropiophenone (1.94 g).

$^1$H-NMR (90 MHz, $CDCl_3$) δ 1.17 (t, J = 7.3 Hz, 3H), 2.97 (q, J = 7.3 Hz, 2H), 3.75 (s, 3H), 3.90 (s, 3H), .96 (s, 3H), 6.70 (d, J = 9.0 Hz, 1H), 7.43 (d, J = 9.0 Hz, 1H)
FAB-MS m/z = 225 ($M^+$ + 1)

Process 2

2',3',4'-Trimethoxypropiophenone (1.12 g) obtained in the above Process 1 and indole-3-carboxaldehyde (0.73 g) were dissolved in ethanol (10 ml), and piperidine (0.43 g) was added thereto, followed by heating under reflux for 72 hours. The precipitated crystals were collected by filtration, and the obtained crude crystals were recrystallized from ethanol to give Compound 24 (0.49 g).

$^1$H-NMR (270 MHz, $CDCl_3$) $\delta$ 2.29 (d, J = 1.0 Hz, 3H), 3.86 (s, 3H), 3.92 (s, 3H), 3.94 (s, 3H), 6.74 (d, J = 8.6 Hz, 1H), 7.06 (d, J = 8.6 Hz, 1H), 7.15 (m, 1H), 7.25 (m, 1H), 7.41 (brd, J = 7.9 Hz, 1H), 7.48 (brd, J = 7.9 Hz, 1H), 7.58 (brs, 1H), 7.62 (d, J = 3.0 Hz, 1H), 8.74 (brs, 1H)
EI-MS m/z = 351 ($M^+$)

| Elemental analysis: $C_{21}H_{21}NO_4$ | | | |
|---|---|---|---|
| Calcd.(%): | C, 71.78; | H, 6.02; | N, 3.99 |
| Found (%): | C, 71.78; | H, 6.19; | N, 3.90 |

<u>Industrial Applicability</u>

According to the present invention, there can be provided propenone derivatives having an excellent antitumor activity.

**Claims**

1. A propenone derivatives represented by the following formula (I):

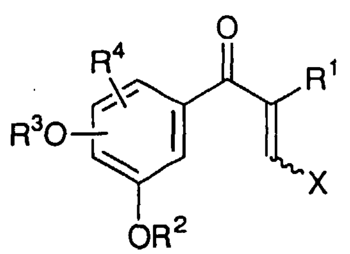

(I)

wherein $R^1$ represents hydrogen or lower alkyl; $R^2$ and $R^3$ independently represent hydrogen, lower alkyl, or substituted or unsubstituted aralkyl, or alternatively $R^2$ and $R^3$ are combined to form substituted or unsubstituted methylene or ethylene; $R^4$ represents hydrogen, hydroxy, lower alkyl, substituted or unsubstituted aralkyl, lower alkoxy, substituted or unsubstituted aralkyloxy, or halogen; and X represents substituted or unsubstituted indolyl, with the proviso that when $OR^3$ is on the position 2 or position 6 of the benzene ring, $R^3$ is not hydrogen; that when $R^4$ is on the position 2 or position 6 of the benzene ring, $R^4$ is not hydroxy; and that the combination of $OR^2$, $OR^3$, and $R^4$ is not 3,4-dimethoxy or 3,4,5-trimethoxy; or pharmaceutically acceptable salts thereof.

2. A propenone derivative or a pharmaceutically acceptable salt thereof according to Claim 1, wherein $R^2$ and $R^3$ are combined to form substituted or unsubstituted methylene or ethylene.

3. A propenone derivative or a pharmaceutically acceptable salt thereof according to Claim 1, wherein $OR^3$ represents lower alkoxy, or substituted or unsubstituted aralkyloxy on the position 2 or position 6 of the benzene ring.

4. A propenone derivative or a pharmaceutically acceptable salt thereof according to Claim 1, wherein $OR^3$ and $R^4$ are on the position 4 or position 5 of the benzene ring.

5. A propenone derivative or a pharmaceutically acceptable salt thereof according to Claims 1 to 4, wherein $R^4$ rep-

resents lower alkyl, substituted or unsubstituted aralkyl, or halogen.

6. A pharmaceutical composition comprising a propenone derivative or a pharmaceutically acceptable salt thereof according to Claims 1 to 5.

## INTERNATIONAL SEARCH REPORT

International application No.
PCT/JP96/01156

**A. CLASSIFICATION OF SUBJECT MATTER**

Int. $Cl^6$ C07D209/12, 405/10, A61K31/40

According to International Patent Classification (IPC) or to both national classification and IPC

**B. FIELDS SEARCHED**

Minimum documentation searched (classification system followed by classification symbols)

Int. $Cl^6$ C07D209/12, 405/10, A61K31/40

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| P,A | WO, 95/19169, A2 (Sugen Inc. et al.), July 20, 1995 (20. 07. 95), Full descriptions & AU, 9515633, A | 1 - 6 |
| Y | Cancer Research, 54(23), (1994), p. 6106-14, Marina Kovalenko, et al., "Selective Platelet-derived Growth Factor Receptor Kinase Blockers Reverse sis-Transformation" | 1 - 6 |
| Y | US, 4904697, A (Merrell Dow Pharmaceuticals Inc.), February 27, 1990 (27. 02. 90), Full descriptions & EP, 288794, A & AU, 8814164, A & ZA, 8802391, A | 1 - 6 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| July 23, 1996 (23. 07. 96) | July 30, 1996 (30. 07. 96) |
| Name and mailing address of the ISA/ Japanese Patent Office | Authorized officer |
| Facsimile No. | Telephone No. |

Form PCT/ISA/210 (second sheet) (July 1992)